(19) Europäisches Patentamt
European Patent Office
Office européen des brevets

(11) Veröffentlichungsnummer : **0 068 144**
**B1**

(12) **EUROPÄISCHE PATENTSCHRIFT**

(45) Veröffentlichungstag der Patentschrift : **30.10.85**

(21) Anmeldenummer : **82104542.4**

(22) Anmeldetag : **25.05.82**

(51) Int. Cl.⁴ : **C 07 D249/08**, C 07 D233/58, C 07 D235/06, C 07 D231/12, C 07 D233/68, C 07 D233/91, C 07 D233/64, A 61 K 31/41, A 61 K 31/415

(54) **1,1-Diphenyl-2-triazolyl-ethane, ihre Herstellung und ihre Verwendung als Fungizide.**

(30) Priorität : **01.06.81 DE 3121676**

(43) Veröffentlichungstag der Anmeldung : **05.01.83 Patentblatt 83/01**

(45) Bekanntmachung des Hinweises auf die Patenterteilung : **30.10.85 Patentblatt 85/44**

(84) Benannte Vertragsstaaten : **AT BE CH DE FR GB IT LI NL SE**

(56) Entgegenhaltungen :
**EP-A- 0 017 080**
**EP-A- 0 025 882**
**EP-A- 0 037 049**
**DE-A- 2 604 047**
**DE-A- 2 851 059**
**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt am Main 80 (DE)**

(72) Erfinder : **Hertenstein, Ulrich, Dr.**
**Goethestrasse 3**
**D-8901 Garblingen (DE)**
Erfinder : **Mildenberger, Hilmar, Dr.**
**Fasanenstrasse 24**
**D-6233 Kelkheim (Taunus) (DE)**
Erfinder : **Sachse, Burkhard, Dr.**
**An der Ziegelei 30**
**D-6233 Kelkheim (Taunus) (DE)**
Erfinder : **Hartz, Peter, Dr.**
**An der Ziegelei 28**
**D-6233 Kelkheim (Taunus) (DE)**

**EP 0 068 144 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 1,1-Diphenyl-2-triazolyl-ethane, ein Verfahren zur Herstellung dieser Substanzklasse sowie ihre Verwendung als Fungizide.

Es ist bekannt, daß Verbindungen aus der Klasse der 1,1-Diaryl-2-imidazolyl-ethane fungizide Eigenschaften besitzen (US-A 4 115 578, DE-A 26 04 047). Über ihre Aktivität, insbesondere bei niedrigen Aufwandmengen, ihre Wirkungsbreite sowie den Wirkungsschwerpunkt liegen jedoch keine näheren Angaben vor.

Eine Vielzahl von 1,1-Diaryl-2- oder -3-triazolyl-alkanen, die meistens in 1-Stellung noch eine Cyanogruppe tragen, ist aus der BE-A 867 245 bekannt. Zu den in der Beschreibung genannten Verbindungen zählt auch das 1-(2,4-Dichlorphenyl)-1-(4-chlorphenyl)-2-(1,2,4-triazol-1-yl)-ethan, das aber — obwohl strukturverwandt — weniger wirksam als vergleichbare erfindungsgemäße Verbindungen ist.

Es wurde gefunden, daß bestimmte, bisher nicht beschriebene 1,1-Diphenyl-2-triazolyl-ethane — verglichen mit den bereits bekannten — überraschenderweise eine erheblich höhere fungizide Potenz — insbesondere gegen Rost und Mehltau — aufweisen.

Gegenstand der Erfindung sind daher die 1,1-Diphenyl-2-triazolyl-ethane der allgemeinen Formel (I)

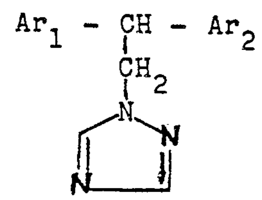

(I)

in welcher $Ar_1$ und $Ar_2$ für gleiche oder verschiedene Reste stehen, die die Bedeutung von Resten der Formel (II) haben,

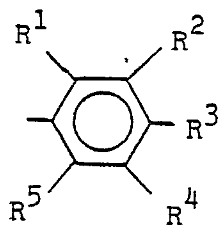

(II)

wobei in dieser Formel $R^1$ bis $R^5$ gleich oder verschieden sind und für Wasserstoff, Halogen, $CF_3$, ($C_1$-$C_8$)Alkyl oder ($C_1$-$C_6$)Alkoxy steht, wobei die Verbindung, für die $R^1$, $R^3$ oder $R^5$, $R^3$ = Chlor und $R^2$, $R^4$, $R^5$ oder $R^1$, $R^2$, $R^4$ = H im Rest $Ar_1$ oder $Ar_2$ bedeuten und $R^1$, $R^2$, $R^4$, $R^5$ = H und $R^3$ = Chlor im jeweils anderen Rest $Ar_2$ oder $Ar_1$ bedeuten, ausgenommen ist, sowie ihre Salze, Komplexsalze und Quaternierungsprodukte.

Die neuen Verbindungen werden aus, eine alkoholische OH-Gruppe tragenden Verbindungen der Formel (V),

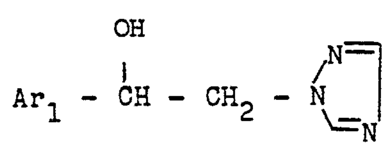

(V)

in welcher $Ar_1$ die oben angegebenen Bedeutungen besitzen, durch Reaktion mit Arylkohlenwasserstoffen der Formel (VI)

$$H—Ar_2 \qquad (VI),$$

in der $Ar_2$ einer der vorstehend angegebenen Reste ist, erhalten.

Man arbeitet im Temperaturbereich von − 15 bis + 150 °C, vorzugsweise von − 10 bis 110 °C, in Gegenwart eines sauren Katalysators, gegebenenfalls bei Anwesenheit eines Verdünnungsmittels. Unter sauren Katalysatoren sind hierbei die üblichen Friedel-Crafte-Katalysatoren zu verstehen, wie sie — ebenso wie geeignete Verdünnungsmittel — z. B. im Houben-Weyl, Methoden der org. Chemie, Bd. 7/2a, S. 17 bis 21, beschrieben sind. Bevorzugt werden Tetrachlorethan und Aluminiumtrichlorid. Vom Katalysator setzt man 1,1 bis 2,5 Äquivalente, vom Arylkohlenwasserstoff H—$Ar_2$ zumindest 1 Äquivalent, bezogen auf die Verbindung der Formel (V), ein.

Für die Herstellung der 1,1-Diphenyl-2-triazolyl-ethane geeignete Ausgangsverbindungen der Formel (V) sind beispielsweise :

1-(2,4-Dichlorphenyl)-1-hydroxy-2-(1,2,4-triazol-1-yl)-ethan, 1-(2,4-Dichlorphenyl)-1-hydroxy-2-(imidazolyl-1-yl)-ethan, 1-(3,4-Dichlorphenyl)-1-hydroxy-2-(1,2,4-triazol-1-yl)-ethan, 1-(2,5-Dichlorphenyl)-1-hydroxy-2-(1,2,4-triazol-1-yl)-ethan, 1-(2-Methyl-4-chlorphenyl)-1-hydroxy-2-(1,2,4-triazol-1-yl)-ethan und 1-(4-Methoxyphenyl)-1-hydroxy-2-(1,2,4-triazol-1-yl)-ethan.

Unter Aromaten der Formel H—$Ar_2$ (VI) werden z. B. verstanden :
Chlorbenzol, Brombenzol, 1,3-Dichlorbenzol, 1,2-Dichlorbenzol, 1,4-Dichlorbenzol, Toluol, Xylol ; Mesitylen, 3-Chlortoluol, 4-Chlortoluol and Anisol.

Bei der Synthese geht man im einzelnen so vor, daß man die Verbindungen der Formel (V), den Arylkohlenwasserstoff H—$Ar_2$(VI) und gegebenenfalls ein Verdünnungsmittel vorlegt und den Katalysator bei ca. – 10 °C langsam zudosiert. Man läßt innerhalb 30 min auf Raumtemperatur kommen und heizt dann bis zur beginnenden Gasentwicklung auf. Ist diese beendet, so heizt man 30 min nach, läßt auf Raumtemperatur abkühlen, gießt auf Eis, macht mit 50 %iger NaOH unter Eiskühlung stark alkalisch und arbeitet dann die organische Phase wie üblich auf.

Der Reaktionsverlauf sei nachstehend am Beispiel der Umsetzung von 1,3-Dichlorbenzol mit 1-(4-Chlorphenyl)-1-hydroxy-2-(1,2,4-triazol-1-yl)-ethan zu dem nicht erfindungsgemäß beanspruchten Produkt (III) aufgezeigt :

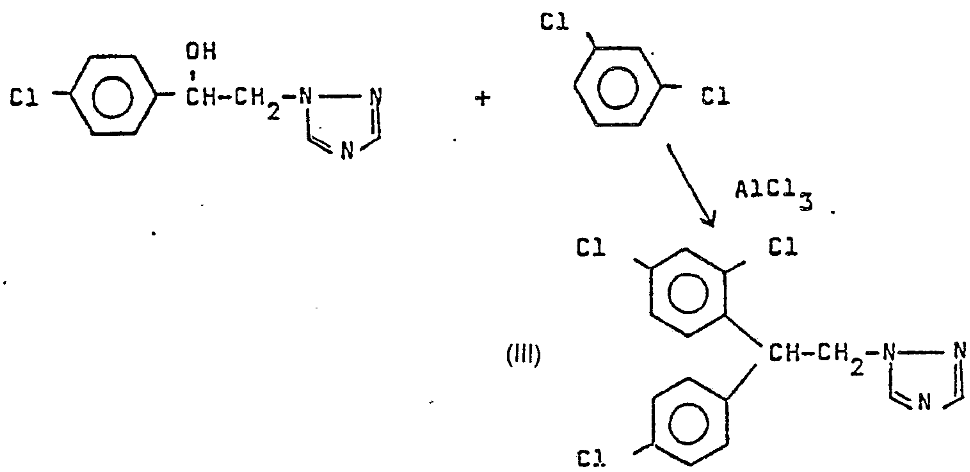

(III)

Dieser Reaktionsverlauf ist überraschend und war nicht vorhersehbar, da unter den Verfahrensbedingungen mit einer Wasserabspaltung zum ω-(1,2,4-Triazol-1-yl)-styrol zu rechnen war, wie dies bei 1-Phenylethanolen bekannt ist [Journ. Am. Chem. Soc. *73* (1951), S. 455 ; Journ. Chem. Soc. *87*, S. 672], und auch eine Triazolabspaltung oder eine Polymerisation denkbar sind.

Die erfindungsgemäßen Verbindungen der Formel (I) können in R- und S-Form auftreten. Als basische Verbindungen sind sie ferner zur Bildung von Salzen, Komplexsalzen und Quarternisierungsprodukten befähigt. Genannt seien Salze organischer und anorganischer Säuren, wie z. B. Acetate, Fumarate, Oxalate, Benzoate, Nitrate, Bromide, Chloride, Sulfate, Salze der Naphthalinsulfonsäuren, Komplexe mit Metallen der Gruppe 1b, 2b, 4b, 8b des Periodensystems, etwa Kupfer, Zink und Zinn und Quaternisierungsprodukte mit Alkyl- und Phenacylhalogeniden. Die Herstellung solcher Verbindungen erfolgt nach den allgemein üblichen Methoden.

Die erfindungsgemäßen Verbindungen zeichnen sich durch eine hervorragende fungizide Wirkung aus, insbesondere z. B. bei der Anwendung im Pflanzenschutz. Dabei lassen sich bereits in das pflanzliche Gewebe eingedrungene pilzliche Krankheitserreger erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt z. B. neben verschiedenen Rostarten, Phytophthora infestans, Plasmopara viticola und Piricularia oryzae, vor allem aber Echte Mehltaupilze im Obst-, Gemüse-, Getreide- und Zierpflanzenbau. Besonders hervorzuheben ist die ausgezeichnete Wirkung der Verbindungen gegen Mehltauarten, die gegen Benzimidazolderivate (z. B. Benomyl, Carbendazim) resistent sind.

Die Substanzen eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden : Alkylarylsulfonsaure Calciumsalze wie Cadodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitan-fettsäureester oder Polyoxethylen-sorbitester.

Stäubemittel werden durch Vermahlen des Wirkstoffes mit fein verteilten, festen Stoffen, z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise — gewünschtenfalls in Mischung mit Düngemitteln — granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen wie Insektiziden, Akariziden, Herbiziden, Düngemitteln, Wachstumsregulatoren oder weiteren Fungiziden sind gegebenenfalls möglich, wobei u. U. auch synergistische Wirkungssteigerungen erzielt werden können.

Im folgenden seien einige Formulierungsbeispiele angeführt :

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispergierbare, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (z. B. ®Triton × 207 der Rohm & Haas Co.), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

## Herstellungsbeispiele

### Beispiel 1

1,1-Diphenyl-2-(1,2,4-triazol-1-yl)-ethan

(Siehe Figur Seite 5 f.)

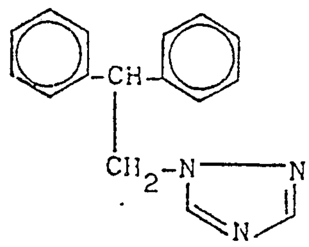

26,7 g (0,2 Mol) $AlCl_3$ gibt man portionsweise bei − 10 °C zu einer Lösung von 18,9 g (0,1 Mol) 1-Phenyl-2-(1,2,4-triazol-1-yl)-ethanol in 50 ml Benzol und 50 ml 1,2-Dichlorethan. Danach erwärmt man innerhalb 30 min. auf Raumtemperatur und heizt dann bis 80 °C auf, bis keine Gasentwicklung mehr beobachtet wird. Man gießt auf 200 ml Eiswasser, trennt die organische Phase ab, engt ein und reibt den Rückstand mit Diisopropylether an Ausbeute 21,4 g (86 %), Smp. 107-108 °C.

$C_{16}H_{15}N_3$ ber. C 77,1 % H 6,0 % N 16,9 %
gef. C 77,0 % H 5,9 % N 17,2 %

$^1$H-NMR ($CDCl_3$, TMSint.) : δ = 4,40-4,82 (m, 3H, CH—$CH_2$)
7,00-7,35 (m, 10H, arom.)
7,55 (s, 1H, Azol)
7,88 ppm (s, 1H, Azol)

Beispiel 2

1-(4-Methylphenyl)-1-phenyl-2-(1,2,4-triazol-1-yl)-ethan

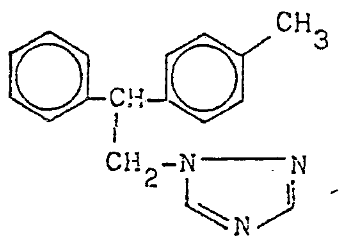

20 g (0,15 Mol) $AlCl_3$ gibt man portionsweise bei − 10 °C zu einer Lösung von 18,9 g (0,1 Mol) 1-Phenyl-2-(1,2,4-triazol-1-yl)-ethanol in 50 ml Toluol und 50 ml 1,2-Dichlorethan. Danach heizt man auf 80 °C auf, bis keine Gasentwicklung mehr beobachtet wird. Man gießt auf 200 ml Eiswasser, trennt die organische Phase ab, engt diese ein und reibt den Rückstand mit Diisopropylether an.
Ausbeute 23,8 g (90 %), Smp. 98-100 °C.

$C_{17}H_{17}N_3$ ber. C 77,6 % H 6,5 % N 16,0 %
gef. C 77,3 % H 6,5 % N 15,9 %
$^1$H-NMR ($CDCl_3$, TMSint.) : = 2,26 (s, 3H, $CH_3$)
4,38-4,80 (m, 3H, CH—$CH_2$)
6,95-7,25 (m, 9H, arom.)
7,50 (s, 1H, Azol)
7,85 ppm (s, 1H, Azol)

Beispiele 3 bis 16

Nach der Arbeitsweise des Beispieles 1 wurden die in nachstehender Tabelle aufgeführten Verbindungen der Formel (IV)

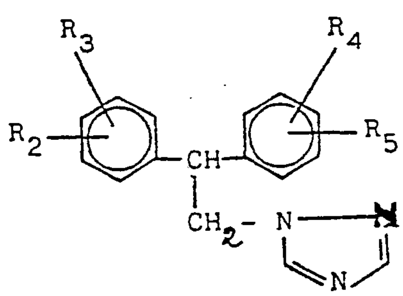 (IV)

hergestellt :

| Bsp. Nr. | Ausgangsmaterialien | | | | physikal. Kenndaten<br>Kp (°C) bei Druck (mbar) bzw. Schmelzpunkt |
|---|---|---|---|---|---|
| | 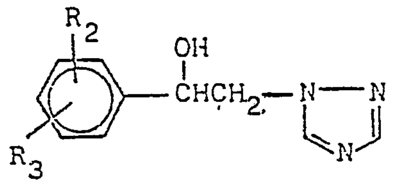 | | 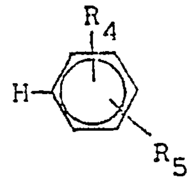 | | |
| | $R_2$ | $R_3$ | $R_4$ | $R_5$ | |
| 3 | H | H | H | 4-$C(CH_3)_3$ | 187-98/0,001 |
| 4 | H | H | H | 4-$OCH_3$ | 192-205/0,05 |
| 5 | H | H | 2-$CH_3$ | 4-$CH_3$ | 160-70/0,0007 |
| 6 | H | H | 2-$CH_3$ | 5-$CH_3$ | 175-82/0,001 |
| 7 | H | H | H | 4-Br | 208/0,005 |
| 8 | H | H | H | 4-Cl | 175-9/0,002 |
| 9 | H | 4-Cl | H | 4-Br | 208-19/0,004 |
| 10 | H | 4-Cl | H | 4-Cl | 129-33 |
| 11 | H | H | 2-Cl | 4-Cl | 178-88/0,0001 |
| 12 | H | 4-Cl | 2-$CH_3$ | 4-$CH_3$ | 215-222/0,005 |
| 13 | H | 4-Cl | 2-$CH_3$ | 5-$CH_3$ | 180-90/0,0001 |
| 14 | H | 4-Cl | H | 4-$CH_3$ | 110-3 |
| 15 | H | 4-Cl | H | 4-OH | 58-60 |
| 16 | H | 4-Cl | H | 4-$OCH_3$ | 170-8/0,0006 |

## Biologische Beispiele

In den folgenden Beispielen stehen die Buchstaben A, B, C und D für die nachstehend genannten handelsüblichen Vergleichsmittel mit bekannten fungiziden Wirkstoffen :

A : Methyl-1-(butylcarbamoyl-2-benzimidazolcarbamat) (Benomyl)
B : N-Tridecyl-2,6-dimethyl-morpholin (Tridemorph)
C : 5-Methyl-1,2,4-triazolo-[3,4-b]-benzothiazol (Tricyclazol)
D : 5,6-Dihydro-2-methyl-1,4-oxathiin-3-carboxanilid-4,4-dioxid

### Beispiel I

Weizenpflanzen werden im 3-Blattstadium mit Konidien des Weizenmehltaus (Erysiphe graminis) stark inokuliert und in einem Gewächshaus bei 20 °C und einer relativen Luftfeuchte von 90-95 % aufgestellt. 3 Tage nach Inokulation werden die Pflanzen mit den in Tabelle I aufgeführten Verbindungen in den Wirkstoffkonzentrationen von 500, 250, 125, 60 und 30 mg/Liter Spritzbrühe tropfnaß gespritzt. Als Vergleich wird das Vergleichsmittel B in analoger Weise eingesetzt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle I zusammengefaßt.

Tabelle I

| Verbindung gemäß Beispiel Nr. | Mit Weizenmehltau befallene Blattfläche in % bei mg Wirkstoff/l Spritzbrühe | | | | |
|---|---|---|---|---|---|
| | 500 | 250 | 125 | 60 | 30 |
| 1 | 0 | 0 | 0 | 0 | 0 |
| 2 | 0 | 0 | 0 | 0 | 0 |
| 3 | 0 | 0 | 0 | 0 | 0 |
| 4 | 0 | 0 | 0 | 0 | 0 |
| 5 | 0 | 0 | 0 | 0 | 0 |
| 6 | 0 | 0 | 0 | 0 | 0 |
| 7 | 0 | 0 | 0 | 0 | 0 |
| 8 | 0 | 0 | 0 | 0 | 0 |
| 9 | 0 | 0 | 0 | 0 | 0 |
| 10 | 0 | 0 | 0 | 0 | 0 |

Tabelle I (Fortsetzung)

| Verbindung gemäß Beispiel Nr. | Mit Weizenmehltau befallene Blattfläche in % bei mg Wirkstoff/l Spritzbrühe 500 | 250 | 125 | 60 | 30 |
|---|---|---|---|---|---|
| 11 | 0 | 0 | 0 | 0 | 0 |
| 12 | 0 | 0 | 0 | 0 | 0 |
| 13 | 0 | 0 | 0 | 0 | 0 |
| 14 | 0 | 0 | 0 | 0 | 0 |
| 15 | 0 | 0 | 0 | 0-3 | 3 |
| 16 | 0 | 0 | 0 | 0-3 | 3 |
| Vergleichsmittel B | 3 Phytotoxis | 5 Phyto toxis | 10 | 15 | 25 |
| unbehandelte, infiz. Pflanzen | | 100 | | | |

Beispiel II

Reispflanzen wurden im 4-Blattstadium mit den in Tabelle II angegebenen Verbindungen in Konzentrationen von 500, 250 und 120 mg Wirkstoff pro Liter Spritzbrühe tropfnaß gespritzt. Nach dem Abtrocknen des Spritzbelages wurden die Pflanzen mit einer Sporensuspension von Piricularia oryzae gleichmäßig besprüht und für 48 h in eine dunkel gehaltene Klimakammer mit 25 °C und 100 % relativer Luftfeuchte gestellt. Anschließend wurden die Pflanzen in einem Gewächshus bei 25 °C und 85 % relativer Luftfeuchte gehalten und 14 Tage nach Inokulation auf Befall mit Piricularia oryzae untersucht. Der Befallsgrad wurde ausgedrückt in % befallener Blattfläche, bezogen auf unbehandelte, infizierte Kontrollpflanzen (= 100 % Befall).

Tabelle II

| Verbindung gemäß Beispiel Nr. | % befallene Blattfläche bei mg Wirkstoff/Liter Spritzbrühe 500 | 250 | 120 |
|---|---|---|---|
| 1 | 0 | 0 | 0-3 |
| 2 | 0 | 0 | 0-3 |
| 3 | 0 | 0 | 0 |
| 4 | 0 | 0 | 0-3 |
| 5 | 0 | 0 | 0-3 |
| 6 | 0 | 0 | 0 |
| 10 | 0 | 0 | 0-3 |
| 11 | 0 | 0 | 0 |
| 12 | 0 | 0 | 0 |
| 14 | 0 | 0 | 0-3 |
| 16 | 0 | 0 | 0-3 |
| Vergleichsmittel C | 0 | 3 | 5 |
| unbehandelte, infiz. Pflanzen | | 100 | |

Beispiel III

Weizenpflanzen wurden mit den in Tabelle III genannten Verbindungen in den Aufwandmengen von 500, 250, 120 und 60 mg/Liter Spritzbrühe tropfnaß behandelt. Nach dem Abtrocknen des Wirkstoffbelages wurden die Pflanzen mit Sporen des Weizenbraunrostes (Puccinia triticina) stark inokuliert und für 24 Stunden in eine Klimakammer mit 100 % rel. Luftfeuchte von 20 °C gestellt. Anschließend kamen die Pflanzen in ein Gewächshaus und wurden hier 14 Tage nach Inokulation auf Befall mit Weizenbraunrost untersucht. Als Vergleichsmittel diente D.

(Siehe Tabelle III Seite 8 f.)

Tabelle III

| Verbindung gemäß Beispiel Nr. | mit Weizenbraunrost befallene Blattfläche in % bei mg Wirkstoff pro Liter Spritzbrühe 500 | 250 | 120 | 60 |
|---|---|---|---|---|
| 8 | 0 | 0 | 0 | 0 |
| 10 | 0 | 0 | 0 | 0 |
| 13 | 0 | 0 | 0 | 0 |
| 14 | 0 | 0 | 0 | 0 |
| 15 | 0 | 0 | 0-3 | 3 |
| 16 | 0 | 0 | 0 | 0 |
| 1 | 0 | 0 | 0 | 0 |
| 12 | 0 | 0 | 0 | 0-3 |
| 11 | 0 | 0 | 0 | 0 |
| 2 | 0 | 0 | 0-3 | 3 |
| 3 | 0 | 0 | 0-3 | 3 |
| 4 | 0 | 0 | 0-3 | 3 |
| 5 | 0 | 0 | 0-3 | 3 |
| 6 | 0 | 0 | 0-3 | 3 |
| 7 | 0 | 0 | 0 | 0 |
| 9 | 0 | 0 | 0 | 0 |
| Vergleichsmittel D | 5 | 10 | 15 | 35 |
| unbehandelte, infiz. Pflanzen | | 100 | | |

Als technische Biozide zeigten einige Verbindungen ebenfalls eine gute, breit wirksame fungizide und z. T. auch bakterizide Wirkung, hier insbesondere gegen Bacillus subtilis :

Beispiel IV

Mycelstücke (Ø 0,5 cm) des Pilzes Poria monticola wurden in Petrischalen auf Nährboden (Biomalz-Agar für Pilze) im Zentrum aufgebracht ; dem Agar waren zuvor im flüssigen Zustand die beanspruchten Verbindungen in den in Tabelle IV angegebenen Konzentrationen zugesetzt worden. 8 Tage nach der Beimpfung der Platten wurde der Durchmesser des Pilzmycels auf dem Agar ausgemessen und die durch die Präparate hervorgerufene Wachstumshemmüng ausgedrückt in Prozent, bezogen auf die Kontrolle (= beimpfter Agar ohne Wirkstoffzusatz = 0 % Hemmung).

Tabelle IV

| Verbindungen gemäß Beispiel | Hemmung von Poria monticola in % bei mg Wirkstoff/Liter Agar 100 | 50 | 10 | 5 | 1 | 0,5 |
|---|---|---|---|---|---|---|
| 1 | 100 | 100 | 80 | 50 | 0 | |
| 2 | 100 | 100 | 80 | 50 | 0 | |
| 3 | 100 | 100 | 50 | 50 | 0 | |
| 4 | 100 | 100 | 50 | 0 | 0 | |
| 5 | 100 | 100 | 80 | 80 | 0 | |
| 6 | 100 | 100 | 100 | 100 | 100 | 50 |
| 7 | 100 | 100 | 100 | 100 | 100 | 50 |
| 8 | 100 | 100 | 100 | 100 | 50 | 50 |
| 9 | 100 | 100 | 100 | 80 | 0 | |
| 10 | 100 | 100 | 100 | 100 | 80 | 50 |
| 11 | 100 | 100 | 100 | 100 | 100 | 80 |
| 12 | 100 | 100 | 100 | 80 | 0 | |
| 13 | 100 | 100 | 100 | 100 | 80 | 50 |
| 14 | 100 | 100 | 100 | 100 | 80 | 50 |
| 16 | 100 | 100 | 100 | 80 | 0 | |

Beispiel V

Jeweils 0,02 ml einer Sporensuspension von Ulocladium consortiale, Aureobasidium pullulans und

Aspergillus niger wurden in Petrischalen auf Nährböden (Biomalz-Agar für Pilze) tropfenförmig aufgebracht ; dem Agar waren zuvor im flüssigen Zustand die beanspruchten Verbindungen in den in Tabelle V angegebenen Konzentrationen zugesetzt worden. 6 Tge nach der Beimpfung der Platten wurde der Durchmesser der Pilzkolonien auf dem Agar ausgemessen und die durch die Präparate hervorgerufene Wachstumshemmung ausgedrückt in Prozent, bezogen auf die Kontrolle (= beimpfter Agar ohne Wirkstoffzusatz = 0 % Hemmung).

Tabelle V

| Verbindungen gemäß Beispiel | Hemmung von Ulocladium consortiale, Aureobasidium pullulans und Aspergillus niger in % bei mg Wirkstoff/Liter Agar | | | | | | |
|---|---|---|---|---|---|---|---|
| | | 100 | 50 | 10 | 5 | 1 | 0,5 |
| 5 | Uc | 100 | 100 | 80 | 50 | | |
| | Ap | 100 | 100 | 50 | | | |
| | An | 100 | 50 | 0 | | | |
| 6 | Uc | 100 | 80 | 50 | | | |
| | Ap | 100 | 100 | 0 | | | |
| | An | 80 | 50 | 0 | | | |
| 7 | Uc | 100 | 100 | 80 | 50 | | |
| | Ap | 100 | 100 | 80 | 50 | | |
| | An | 100 | 100 | 50 | | | |
| 8 | Uc | 100 | 100 | 80 | 80 | | |
| | Ap | 100 | 100 | 80 | 80 | | |
| | An | 100 | 100 | 80 | 80 | | |
| 9 | Uc | 100 | 100 | 80 | 50 | | |
| | Ap | 100 | 100 | 100 | 80 | | |
| | An | 80 | 50 | 0 | | | |
| 10 | Uc | 100 | 100 | 100 | 50 | | |
| | Ap | 100 | 100 | 100 | 80 | 50 | 50 |
| | An | 100 | 100 | 80 | 50 | | |
| 11 | Uc | 100 | 100 | 100 | 100 | 80 | 80 |
| | Ap | 100 | 100 | 100 | 100 | 80 | 80 |
| | An | 100 | 80 | 50 | 0 | | |
| 12 | Uc | 100 | 100 | 80 | 80 | | |
| | Ap | 100 | 80 | 50 | 50 | | |
| | An | 50 | 50 | 0 | | | |
| 13 | Uc | 100 | 100 | 80 | 80 | | |
| | Ap | 80 | 80 | 50 | 50 | | |
| | An | 80 | 80 | 50 | 0 | | |
| 14 | Uc | 100 | 100 | 80 | 50 | | |
| | Ap | 100 | 100 | 80 | 50 | | |
| | An | 80 | 80 | 50 | 0 | | |

Beispiel VI

Jeweils 0,02 ml einer Bakteriensuspension von Bacillus subtilis wurden in Petrischalen auf Nährboden (Standard — I — Nähragar für Bakterien) tropfenförmig aufgebracht ; dem Agar waren zuvor in flüssigem Zustand die beanspruchten Verbindungen in den in Tabelle VI angegebenen Konzentrationen zugesetzt worden. Die mit Bakterien beimpften Platten wurden nach 4 Tagen ausgewertet ; hierbei wurde die Hemmung des Wachstums im Vergleich zur Kontrolle (= beimpfter Agar ohne Wirkstoff-Zusatz = 0 % Hemmung) bonitiert.

Tabelle VI

| Verbindungen gemäß Beispiel | Hemmung von Bacillus subtilis in % bei mg Wirkstoff/Liter Agar | | | | |
|---|---|---|---|---|---|
| | 100 | 50 | 10 | 5 | 1 |
| 3 | 100 | 100 | 100 | 100 | 0 |
| 5 | 100 | 100 | 50 | 0 | |
| 6 | 100 | 100 | 50 | 0 | |

Tabelle VI (Fortsetzung)

| Verbindungen gemäß Beispiel | Hemmung von Bacillus subtilis in % bei mg Wirkstoff/Liter Agar | | | | |
|---|---|---|---|---|---|
| | 100 | 50 | 10 | 5 | 1 |
| 9 | 100 | 100 | 50 | 0 | |
| 10 | 100 | 100 | 50 | 0 | |
| 13 | 100 | 100 | 80 | 0 | |
| 15 | 100 | 100 | 80 | 0 | |

**Patentansprüche** (für die Vertragsstaaten : BE, CH, DE, FR, GB, IT, LI, NL, SE)

1. 1,1-Diphenyl-2-triazolyl-ethane der allgemeinen Formel (I)

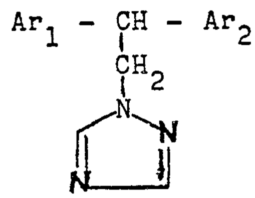

(I)

in welcher $Ar_1$ und $Ar_2$ für gleiche oder verschiedene Reste stehen, die die Bedeutung von Resten der Formel (II) haben,

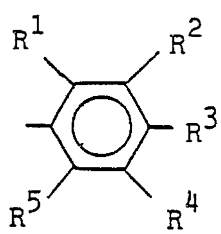

(II)

wobei in dieser Formel $R^1$ bis $R^5$ gleich oder verschieden sind und für Wasserstoff, Halogen, $CF_3$, ($C_1$-$C_8$)Alkyl oder ($C_1$-$C_6$)Alkoxy steht, wobei die Verbindung, für die $R^1$, $R^3$ oder $R^5$, $R^3$ = Chlor und $R^2$, $R^4$, $R^5$ oder $R^1$, $R^2$, $R^4$ = H im Rest $Ar_1$ oder $Ar_2$ bedeuten und $R^1$, $R^2$, $R^4$, $R^5$ = H und $R^3$ = Chlor im jeweils anderen Rest $Ar_2$ oder $Ar_1$ bedeuten, ausgenommen ist, sowie ihre Salze, Komplexsalze und Quaternierungsprodukte.

2. Verbindung der Formel (I), worin $Ar_1$ = Phenyl und $Ar_2$ = 2,4-Dichlorphenyl bedeuten.

3. Verfahren zur Herstellung von Verbindungen der Formel (I) gemäß Ansprüchen 1 und 2, dadurch gekennzeichnet, daß man Verbindungen der Formel (V)

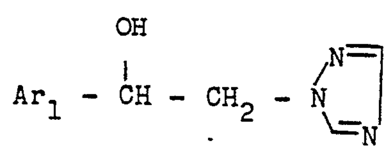

(V)

mit einer Verbindung der Formel (VI)

$$H—Ar_2 \quad (VI),$$

wobei $Ar_1$ und $Ar_2$ die in Anspruch 1 angegebenen Bedeutungen haben, im Temperaturbereich von − 15 °C bis + 150 °C in Gegenwart eines sauren Katalysators und gegebenenfalls in Anwesenheit eines Verdünnungsmittels, einer Friedel-Crafts-Reaktion unterwirft.

4. Verfahren nach Anspruch 3, dadurch gekennzeichnet, daß der saure Katalysator Aluminiumchlorid ist.

5. Fungizide Mittel, die eine Verbindung nach Ansprüchen 1 oder 2 enthalten.

6. Verwendung der Verbindungen nach Ansprüchen 1 oder 2 als Fungizide.

**Patentansprüche** (für den Vertragsstaat AT)

1. Fungizide Mittel mit einem Gehalt an 1,1-Diphenyl-2-triazolyl-ethanen der allgemeinen Formel (I)

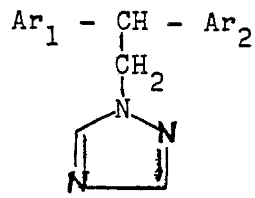

(I)

in welcher $Ar_1$ und $Ar_2$ für gleiche oder verschiedene Reste stehen, die die Bedeutung von Resten der Formel (II) haben,

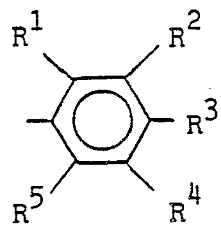

(II)

wobei in dieser Formel $R^1$ bis $R^5$ gleich oder verschieden sind und für Wasserstoff, Halogen, $CF_3$, $(C_1$-$C_8)$Alkyl oder $(C_1$-$C_6)$Alkoxy steht, wobei die Verbindung, für die $R^1$, $R^3$ oder $R^5$, $R^3$ = Chlor und $R^2$, $R^4$, $R^5$ oder $R^1$, $R^2$, $R^4$ = H im Rest $Ar_1$ oder $Ar_2$ bedeuten und $R^1$, $R^2$, $R^4$, $R^5$ = H und $R^3$ = Chlor im jeweils anderen Rest $Ar_2$ oder $Ar_1$ bedeuten, ausgenommen ist, sowie ihren Salzen, Komplexsalzen und Quaternierungsprodukten.

2. Fungizide Mittel, die eine Verbindung der Formel (I) enthalten, worin $Ar_1$ = Phenyl und $Ar_2$ = 2,4-Dichlorphenyl bedeuten.

3. Verfahren zur Herstellung von Verbindungen der Formel (I) gemäß Ansprüchen 1 und 2, dadurch gekennzeichnet, daß man Verbindungen der Formel

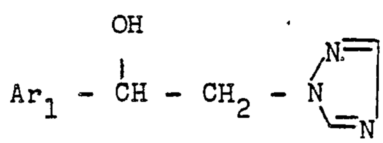

(V)

mit einer Verbindung der Formel (VI)

$$H—Ar_2 \qquad (VI),$$

wobei $Ar_1$ und $Ar_2$ die in Anspruch 1 angegebenen Bedeutungen haben, im Temperaturbereich von − 15 °C bis + 150 °C in Gegenwart eines sauren Katalysators und gegebenenfalls in Anwesenheit eines Verdünnungsmittels, einer Friedel-Crafts-Reaktion unterwirft.

4. Verfahren nach Anspruch 3, dadurch gekennzeichnet, daß der saure Katalysator Aluminiumchlorid ist.

**Claims** (for the Contracting States : BE, CH, DE, FR, GB, IT, LI, NL, SE)

1. 1,1-Diphenyl-2-triazolyl-ethanes of the general formula (I),

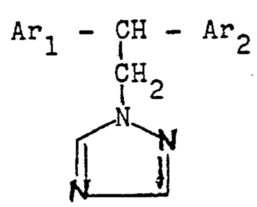

(I)

wherein $Ar_1$ and $Ar_2$ represent identical or different radicals which have the meaning of radicals of the formula (II),

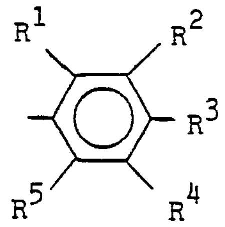

(II)

wherein, in this formula, $R^1$ to $R^5$ are identical or different and represent hydrogen, halogen, $CF_3$, ($C_1$-$C_8$)-alkyl or ($C_1$-$C_6$)alkoxy with the exception of the compound wherein $R^1$, $R^3$ or $R^5$, $R^3$ = chloro and $R^2$, $R^4$, $R^5$ or $R^1$, $R^2$, $R^4$ = H in the radical $Ar_1$ or $Ar_2$ and $R^1$, $R^2$, $R^4$, $R^5$ = H and $R^3$ = chloro in the other radical $Ar_2$ or $Ar_1$, and its salts, complex salts and quaternization products.

2. Compound of the formula I, wherein $Ar_1$ represents phenyl and $Ar_2$ represents 2,4-dichlorophenyl.

3. A process for the preparation of the compounds of formula I according to claims 1 and 2 wherein compounds of the formula (V),

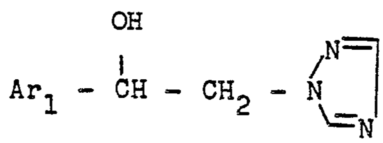

(V)

are subjected to a Friedel-Crafts reaction with a compound of the formula (VI)

$$H—Ar_2 \quad (VI),$$

whereby $Ar_1$ and $Ar_2$ have the meanings given in claim 1, in the temperature range from − 15 °C to + 150 °C in the presence of an acidic catalyst and, if appropriate, in the presence of a diluent.

4. A process according to claim 3 wherein the acidic catalyst is aluminium chloride.

5. Fungicidal preparations which contain a compound according to claims 1 or 2.

6. Use of the compounds according to claims 1 or 2 as fungicides.

**Claims** (for the Contracting State AT)

1. Fungicidal preparations which contain 1,1-Diphenyl-2-triazolyl-ethanes of the general formula (I),

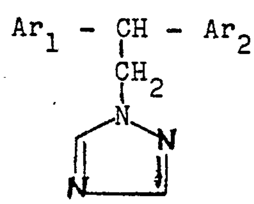

(I)

wherein $Ar_1$ and $Ar_2$ represent identical of different radicals which have the meaning of radicals of the formula (II),

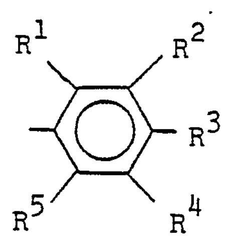

(II)

wherein, in this formula, $R^1$ to $R^5$ are identical or different and represent hydrogen, halogen, $CF_3$, ($C_1$-$C_8$)-alkyl or ($C_1$-$C_6$)alkoxy with the exception of the compound wherein $R^1$, $R^3$ or $R^5$, $R^3$ = chloro and $R^2$, $R^4$, $R^5$ or $R^1$, $R^2$, $R^4$ = H in the radical $Ar_1$ or $Ar_2$ and $R^1$, $R^2$, $R^4$, $R^5$ = H and $R^3$ = chloro in the other radical $Ar_2$ or $Ar_1$, and its salts, complex salts and quaternization products.

2. Fungicidal preparations containing a compound of formula (I) wherein $Ar_1$ = phenyl and $Ar_2$ = 2,4-dichlorophenyl.

3. A process for the preparation of the compounds of formula I according to claims 1 and 2 wherein compounds of the formula (V),

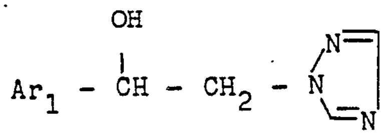

(V)

are subjected to a Friedel-Crafts reaction with a compound of the formula (VI)

$$H—Ar_2 \quad \text{(VI)},$$

whereby $Ar_1$ and $Ar_2$ have the meanings given in claim 1, in the temperature range from − 15 °C to + 150 °C in the presence of an acidic catalyst and, if appropriate, in the presence of a diluent.

4. A process according to claim 3 wherein the acidic catalyst is aluminium chloride.

**Revendications** (pour les Etats contractants : BE, CH, DE, FR, GB, IT, LI, NL, SE)

1. Diphényl-1,1 triazolyl-2 éthanes répondant à la formule générale I :

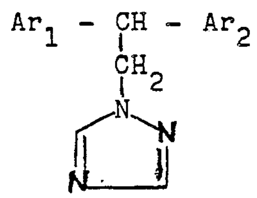

(I)

dans laquelle $Ar_1$ et $Ar_2$ représentent chacun, indépendamment l'un de l'autre, un radical répondant à la formule II :

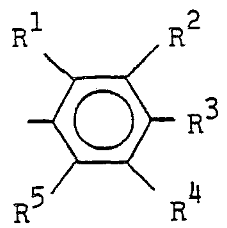

(II)

dans laquelle les symboles $R^1$ à $R^5$ représentent chacun, indépendamment les uns des autres, un atome d'hydrogène ou d'halogène, un radical $—CF_3$, un radical alkyle en $C_1$-$C_8$ ou un radical alcoxy en $C_1$-$C_6$, à l'exception du composé pour lequel $R^1$ et $R^3$ (ou $R^5$ et $R^3$) représentent chacun le chlore tandis que $R^2$, $R^4$ et $R^5$ (ou $R^1$, $R^2$ et $R^4$) représentent chacun l'hydrogène dans le radical $Ar_1$ ou $Ar_2$, et $R^1$, $R^2$, $R^4$ et $R^5$ représentent chacun H tandis que $R^3$ représente le chlore dans l'autre de ces radicaux $Ar_1$ et $Ar_2$. ainsi que leurs sels, leurs sels complexes et leurs produits de quaternisation.

2. Composé de formule I dans lequel $Ar_1$ représente un radical phényle et $Ar_2$ un radical dichloro-2,4 phényle.

3. Procédé de préparation de composés de formule I selon l'une des revendications 1 et 2, procédé caractérisé en ce qu'on soumet à une réaction de Friedel et Crafts un composé de formule V :

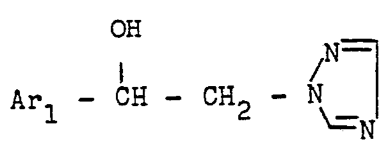

(V)

avec un composé de formule VI ;

$$H—Ar_2 \quad \text{(VI)},$$

formules dans lesquelles $Ar_1$ et $Ar_2$ ont les significations données à la revendication 1, dans un intervalle de température allant de − 15 à + 150 °C, en présence d'un catalyseur acide, et éventuellement, en présence d'un diluant.

4. Procédé selon la revendication 3 caractérisé en ce que le catalyseur acide est le chlorure d'aluminium.

5. Produits fongicides qui contiennent un composé selon l'une des revendications 1 et 2.

6. Application des composés selon l'une des revendications 1 et 2 comme fongicides.

**Revendications** (pour l'Etat contractant AT)

1. Produits fongicides contenant des diphényl-1,1 triazolyl-2 éthanes répondant à la formule générale I :

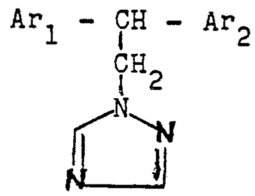

(I)

dans laquelle $Ar_1$ et $Ar_2$ représentent chacun, indépendamment l'un de l'autre, un radical répondant à la formule II :

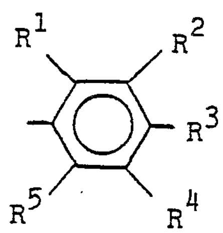

(II)

dans laquelle les symboles $R^1$ à $R^5$ représentent chacun, indépendamment les uns des autres, un atome d'hydrogène ou d'halogène, un radical $—CF_3$, un radical alkyle en $C_1$-$C_8$ ou un radical alcoxy en $C_1$-$C_6$, à l'exception du composé pour lequel $R^1$ et $R^3$ (ou $R^5$ et $R^3$) représentent chacun le chlore tandis que $R^2$, $R^4$ et $R^5$ (ou $R^1$, $R^2$ et $R^4$) représentent chacun l'hydrogène dans le radical $Ar_1$ ou $Ar_2$, et $R^1$, $R^2$, $R^4$ et $R^5$ représentent chacun H tandis que $R^3$ représente le chlore dans l'autre de ces radicaux $Ar_1$ et $Ar_2$. ainsi que leurs sels, leurs sels complexes et leurs produits de quaternisation.

2. Produits fongicides contenant un composé de formule I dans lequel $Ar_1$ représente un radical phényle et $Ar_2$ un radical dichloro-2,4-phényle.

3. Procédé de préparation de composés de formule I selon l'une des revendications 1 et 2, procédé caractérisé en ce qu'on soumet à une réaction de Friedel et Crafts un composé de formule V :

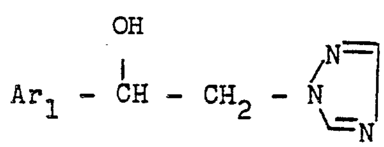

(V)

avec un composé de formule VI :

$$H—Ar_2 \qquad \text{(VI)},$$

formules dans lesquelles $Ar_1$ et $Ar_2$ ont les significations données à la revendication 1, dans un intervalle de température allant de − 15 °C à + 150 °C, en présence d'un catalyseur acide et, le cas échéant, en présence d'un diluant.

4. Procédé selon la revendication 3 caractérisé en ce que le catalyseur acide est le chlorure d'aluminium.